# FASCICULE DE BREVET EUROPEEN

(11) **EP 3 003 195 B1**
(45) Date de publication et mention de la délivrance du brevet: **10.08.2022**
(21) Numéro de dépôt: 14731719.2
(22) Date de dépôt: 26.05.2014
(51) Int. Cl.: A61F 2/00, A61B 50/30

(54) **EMBALLAGE MEDICAL**
MEDIZINISCHE VERPACKUNG
MEDICAL PACKAGING

(30) Priorité: 24.05.2013 FR 1354717
(43) Date de publication de la demande: 13.04.2016
(73) Titulaire: Selenium Medical, 17000 La Rochelle (FR)
(72) Inventeur: RICHART, Olivier, 17580 Le Bois Plage en Ré (FR)
(74) Mandataire: Ipsilon
(86) Numéro de dépôt international: PCT/FR2014/051222
(87) Numéro de publication internationale: WO 2014/188142

(56) Documents cités:
- EP-A1- 1 466 840
- CN-U- 201 923 447
- CN-U- 202 464 396
- DE-U1-202008 009 661
- FR-A1- 2 656 792
- US-A- 2 268 734
- US-A- 4 101 031
- US-A1- 2010 140 124

## Description

La présente invention concerne de manière générale les emballages pour objets médicaux, en particulier pour pièces médicales de préférence stérilisées.

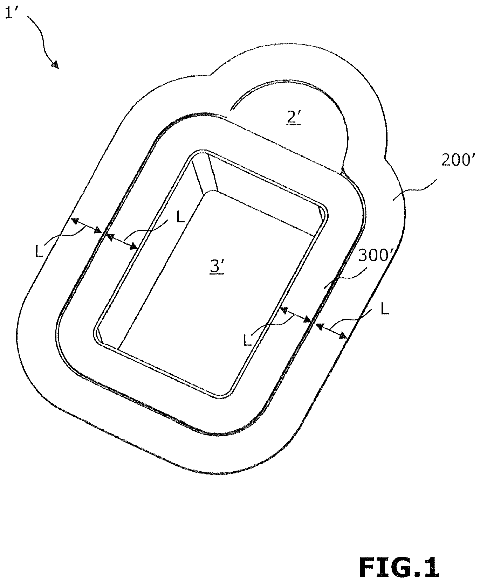

Comme illustré à la figure 1, on connaît de l'état de la technique un ensemble 1' médical comprenant, d'une part, un emballage 3', appelé emballage intérieur, destiné à renfermer une pièce, telle qu'un implant (non représenté), et, d'autre part, un emballage 2' dit extérieur contenant l'emballage 3' intérieur.

Chaque emballage se présente sous la forme d'une coque ouverte dont l'ouverture est refermée par une feuille de scellage pelable (non représentée).

On observe cependant que chaque coque 2', 3' présente autour de son ouverture un rebord 200', 300' externe périphérique qui s'étend perpendiculairement par rapport à l'axe de l'ouverture, c'est-à-dire l'axe orthogonal au plan d'ouverture. C'est sur ce rebord externe périphérique que la feuille de scellage est thermocollée afin de sceller l'emballage formé par la coque correspondante. Autrement dit, le scellage de chaque emballage est réalisé à plat.

Pour répondre à la norme NF EN ISO 868 (en particulier -5) et EN- ISO 11607 (en particulier -1), qui préconise que le chemin le plus court permettant à un liquide de franchir la zone de scellage doit être d'au moins 6 mm, chacun desdits rebords destinés à être recouverts par une feuille de scellage présente une largeur de 6 mm afin d'offrir une zone de scellage périphérique de largeur de 6 mm de sorte que pour chaque emballage un liquide doit parcourir au moins 6 mm à travers la zone de scellage pour sortir de ou pénétrer dans l'emballage.

Il résulte d'une telle conception de chacun de ces emballages 2' 3', que l'encombrement hors-tout de l'emballage est très important. En effet la présence d'un rebord de largeur L= 6mm pour chaque emballage entraine une augmentation de la largeur de l'encombrement de l'ensemble 1 médical de 2*2*6= 24 mm.

Le document CN 202 464 396 U décrit un récipient refermé par un couvercle et équipé d'une bande de ceinturage.

Le document US3485408A décrit deux coques couplées l'une à l'autre par une charnière et présentant des bordures définissant une ouverture d'accès. Les deux coques peuvent être ouvertes ou fermées. Une ouverture et une cale forment un mécanisme de verrouillage.

Le document US 5615770A décrit un emballage support d'implant comprenant une première partie munie de cavités de réception d'implant et une deuxième partie formant couvercle reliée à la première partie par une charnière.

Le document US 2003/214139 décrit un système qui comprend un récipient muni d'un capot de fermeture. Un élément de scellage protecteur est appliqué au capot.

La présente invention a pour but de proposer un nouvel emballage médical et un nouvel ensemble de double emballages médicaux dont l'encombrement est réduit, tout en conservant un scellage de chaque emballage répondant à la norme EN ISO 11607(en particulier -1) & NF EN ISO 868(en particulier -5).

A cet effet, l'invention a pour objet un emballage médical conforme à la revendication 1.

Le scellage le long des bordures d'ouverture des coques de l'emballage permet de réduire l'encombrement de l'emballage tout en assurant un scellage d'étanchéité aux liquides et aux bactéries répondant à la norme EN ISO 11607 (en particulier -1) & NF EN ISO 868 (en particulier -5). En particulier, la conception de l'emballage selon l'invention sous forme de deux coques délimitant entre elles une chambre, permet de réaliser un scellage de la chambre de manière sensiblement circulaire et sur une hauteur réduite de l'emballage.

En effet, contrairement à la solution connue de l'état de la technique, pour obtenir une zone de scellage des coques présentant un plus court chemin d'au moins 6 mm, il n'est pas nécessaire de prévoir un rebord externe de coque qui s'étendrait suivant une direction orthogonale à l'axe d'ouverture de la coque sur une distance de 6 mm. En effet au moins une partie de la zone de scellage peut être définie entre la bande et les coques dans le sens de la hauteur de l'emballage.

Préférentiellement, la zone de contact entre la bande de scellage et les coques forme, le cas échéant avec la zone de contact d'une bordure de coque avec la bordure de l'autre coque, une zone de scellage dont le plus court chemin que doit parcourir un liquide pour traverser cette zone de scellage est au moins égal à 6 millimètres.

Ainsi, une partie de la zone de scellage peut aussi être formée par la zone de contact d'une bordure de coque avec la zone de contact de l'autre bordure de coque de manière à définir avec la bande de scellage une zone de scellage dont la traversée par un liquide nécessite de parcourir au moins 6 millimètres de cette zone de scellage.

On notera que la largeur de la bande de scellage s'étend ainsi sensiblement suivant la hauteur de l'emballage, c'est-à-dire parallèlement à l'axe d'ouverture des coques, ou éventuellement avec une certaine inclinaison, et non pas suivant la largeur de l'emballage, c'est-à-dire non pas suivant une direction orthogonale à l'axe d'ouverture, qui augmenterait l'encombrement de l'emballage de manière importante comme expliqué ci-dessus.

Un tel changement d'orientation de la bande de scellage par rapport à l'état de la technique permet la réduction du volume de l'emballage et de l'approcher au plus près de son contenu.

Une telle conception de chaque emballage à partir de deux coques scellées tout le long de la zone d'ouverture des coques permet également de réduire la surface utilisée de matériau scellable tout en offrant une vision du contenu sur une grande plage angulaire, en particulier sur 360° autour de l'axe de l'emballage, ainsi qu'au dessus et en dessous dudit emballage.

Selon une caractéristique avantageuse de l'invention, les deux coques de l'emballage sont reliées entre elles par une charnière.

La charnière peut être un élément réalisé de manière monobloc avec les deux coques. La charnière peut aussi être un élément rapporté sur les coques. La charnière peut encore être formée par une bande souple, par exemple similaire à la bande de scellage, qui relie l'une à l'autre les bordures des deux coques sur une partie seulement de leur périphérie.

La bande de scellage est appliquée par une de ses extrémités sur la charnière, s'étend autour de l'emballage le long des bordures des coques et revient s'appliquer sur la charnière.

Selon différents mode de réalisation de l'emballage selon l'invention, la bordure d'une coque présente une forme qui s'emboite avec la forme de la bordure de l'autre coque.

Préférentiellement, la bordure de chaque coque est prolongée du côté extérieur de ladite coque par un rebord qui se développe, par rapport à la zone d'ouverture de l'emballage définie entre les bordures des coques, du côté de ladite coque et qui s'étend à écartement de la paroi extérieure de ladite coque pour ménager un espace, appelé espace d'insertion de pièce de contre-appui, entre ledit rebord et la paroi extérieure de ladite coque.

Avantageusement, on peut prévoir que le rebord de chaque coque s'étend sur une hauteur, c'est-à-dire sensiblement parallèlement à l'axe d'ouverture de la coque correspondante, de l'ordre de 6 mm. Préférentiellement la bande de scellage présente une largeur d'au moins 12 mm pour recouvrir chaque rebord. Ces valeurs peuvent être réduites tant que le chemin le plus court pour traverser la zone de scellage reste au moins égal à 6 mm.

Avantageusement, chaque rebord présente une face extérieure, sensiblement plane, qui est soit parallèle à l'axe de l'ouverture de la coque correspondante, soit forme avec l'axe de l'ouverture de ladite coque un angle un angle non nul.

Selon une caractéristique avantageuse de l'emballage selon l'invention, ledit emballage est réalisé en matériau transparent.

Avantageusement, la bande de scellage est appliquée sur l'emballage à chaud et sous pression.

Selon un mode de réalisation préféré, la zone d'ouverture de l'emballage, définie entre les bordures des coques dudit emballage, est plus proche du fond d'une coque que du fond de l'autre coque dudit emballage.

Selon une caractéristique particulière de l'invention, ledit emballage comprend une zone de préhension ménagée à l'extrémité d'une des deux coques dudit emballage.

L'invention concerne également un ensemble comprenant deux emballages médicaux, dont un emballage, dit emballage extérieur, et un emballage, dit emballage intérieur, logé dans l'emballage extérieur, caractérisé en ce que chaque emballage est tel que décrit ci-dessus.

Préférentiellement, en configuration fermée des coques de chaque emballage, l'axe de l'ouverture des coques de l'emballage intérieur est sensiblement parallèle à l'axe de l'ouverture des coques de l'emballage extérieur, la zone d'ouverture de l'emballage extérieur, définie entre les bordures de coques dudit emballage extérieur, et la zone d'ouverture correspondante de l'emballage intérieur étant décalées l'une de l'autre le long de l'axe de l'ouverture des coques de l'emballage extérieur.

L'invention concerne également un procédé de scellage conforme à la revendication 12 ou 13.

L'invention sera bien comprise à la lecture de la description suivante d'exemples de réalisation, en référence aux dessins annexés dans lesquels :
- la figure 1 est une vue d'un ensemble comprenant deux emballages qui est connu de l'état de la technique ;
- la figure 2 est une vue en perspective d'un mode de réalisation d'un ensemble selon l'invention montrant un emballage intérieur scellé à loger dans un emballage extérieur dont les coques sont destinées à être scellées l'une à l'autre;
- la figure 3 est une vue de l'ensemble de la figure 2 à l'état introduit de l'emballage intérieur dans l'emballage extérieur et à l'état scellé dudit emballage extérieur;
- la figure 3A est une vue de détail de la zone référencée III de l'ensemble de la figure 3 ;
- la figure 4 est une vue d'un mode de réalisation particulier d'un emballage selon l'invention ;
- la figure 5 est une vue partielle en coupe axiale de l'emballage de la figure 4 à l'état rapporté de deux pièces de contre appui avant scellage des coques entre elles ;
- la figure 6 est une vue partielle en coupe axiale d'un autre mode de réalisation d'un emballage selon l'invention ;
- la figure 7 est une vue partielle en coupe axiale d'un autre mode de réalisation d'un emballage selon l'invention.

En référence aux figures et comme rappelé ci-dessus, l'invention concerne un ensemble 1 médical, comprenant un emballage 2, dit emballage extérieur, et un emballage 3, dit emballage intérieur, logeable dans l'emballage 2 extérieur.

Chaque emballage 2, 3 est formé à partir de deux coques 21, 22, 31, 32 ouvertes. Autrement dit, chaque coque définit une cavité borgne. Les ouvertures des coques 21, 22, 31, 32 d'un emballage 2, 3 peuvent être ramenées l'une vers l'autre, et de préférence l'une contre l'autre, pour délimiter une chambre. La chambre ainsi définie par les coques 21, 22 de l'emballage 2 extérieur permettent de renfermer l'emballage 3 intérieur 31, 32 dont la chambre formée par les coques correspondantes permet de renfermer un objet médical.

En particulier, l'ensemble 1 est conçu à des fins de préservation du caractère stérile dudit objet et en vue du déballage de l'objet dans des conditions aseptiques ou proches de l'asepsie. Ledit objet peut être par exemple une pièce solide, telle qu'une vis pour chirurgie, ou tout autre type d'objet, en particulier tout autre type d'implant. En outre, ledit objet peut être un liquide ou une poudre. Ledit objet et, de préférence, les différentes parties de l'emballage, sont stérilisés par exemple par rayonnement.

En particulier, chaque coque 21, 22, 31, 32 de chaque emballage 2, 3 présente une bordure 210, 220 qui délimite une ouverture d'accès à l'intérieur de la coque. Les bordures des coques 31, 32 n'apparaissent pas aux figures caractéristique elles sont recouvertes de la bande de scellage 4, mais la description réalisées pour les bordures 210, 220 des coques 21, 22 s'appliquent aussi aux bordures des coques 31, 32.

Chaque emballage 2, 3 présente au moins une configuration dite fermée selon laquelle ladite bordure d'accès d'une coque vient en regard, et de préférence en contact, de la bordure d'accès de l'autre coque pour former une chambre sensiblement fermée. Chaque emballage 2, 3 présente aussi une configuration dite ouverte dans laquelle ladite chambre est ouverte de manière à pouvoir accéder au contenu de ladite chambre. A cet effet, à l'état non scellé, les coques d'un emballage sont déplaçables l'une par rapport à l'autre, par exemple par séparation ou par articulation comme détaillé ci-après.

Chaque bordure d'une coque présente une largeur définie entre le pourtour linéique intérieur de l'ouverture de la coque et le pourtour linéique extérieur de l'ouverture de la coque. Autrement dit, la bordure présente une largeur correspondant à l'épaisseur de la coque au niveau de son embouchure. La bordure d'une coque correspond ainsi à la surface du pourtour de l'embouchure de la coque apte à venir en regard ou en contact de la bordure opposée de l'autre coque.

Chaque bordure s'arrête à la charnière de liaison des coques entre elles lorsqu'une telle charnière est présente comme détaillé ci-après.

Chaque coque 21, 22, 31, 32 d'un emballage présente un axe A2, A3 orthogonal au plan moyen d'ouverture de la coque et passant sensiblement par le centre de ladite ouverture. Cet axe est appelé axe de l'ouverture de la coque. En configuration fermée d'un emballage, l'axe de l'ouverture d'une coque d'un emballage est sensiblement confondu avec l'axe d'ouverture de l'autre coque de l'emballage de sorte qu'il correspond à l'axe de l'emballage ou encore à l'axe de la zone d'ouverture de l'emballage, c'est-à dire à l'axe orthogonal au plan moyen passant entre les bordures des coques de l'emballage en configuration fermée dudit emballage.

On peut prévoir que chaque emballage 2, 3 soit réalisé de manière monobloc. Chaque coque est de préférence thermoformée. En variante, chaque coque peut être réalisée par moulage par exemple par injection. Chaque coque présente une forme générale de révolution autour d'un axe correspondant à l'axe de l'ouverture de la coque. Chaque coque peut prendre d'autre formes, mais de préférence sans angle vifs au niveau de la zone de scellage des coques entre elles pour favoriser la bonne application de la bande de scellage 4 sur ladite zone d'ouverture des coques.

Chaque emballage 2, 3 comprend une bande de scellage 4 qui s'étend, du côté extérieur des coques, sur toute la longueur des bordures 210, 220 des coques de l'emballage et, d'une bordure de coque à l'autre de manière à recouvrir par l'extérieur des coques, la zone d'ouverture des coques de manière à sceller la chambre délimitée par les deux coques. La zone d'ouverture des coques correspond à la zone délimitée entre lesdites bordures de coques. Cette zone d'ouverture peut être formée d'un espace interstitiel ou de la zone de contact entre les bordures suivant que les bordures sont ou non en appui ou contact l'une contre l'autre.

Préférentiellement, la bande de scellage 4 est une bande pelable. Une fois la bande appliquée, celle-ci peut être retirée en tirant dessus pour la peler. On entend par pelable le fait que la bande ne se déchire pas lorsqu'on la retire, tandis que la colle reste sur la zone des coques précédemment scellée.

En variante, on peut prévoir que la bande soit déchirable. Selon un mode de réalisation ladite bande est thermocollante par exemple en matériau synthétique non-tissé fabriquée à partir de fibres de polyéthylène, usuellement vendue sous la marque déposée TYVEK. On peut prévoir de remplacer cette solution de scellage à chaud sous pression par une autre solution de scellage, par exemple à l'aide d'une colle chimique adaptée.

Dans le cas d'une bande thermocollante, la bande de scellage 4 est appliquée sur chaque emballage 2, 3 à chaud et sous pression. La bande de scellage 4 comprend une face munie de colle qui devient active lorsqu'elle est appliquée sur le support à sceller avec une pression et une température donnée.

La bande de scellage 4 recouvre ainsi les bordures, en particulier la périphérie extérieure des bordures des coques, en s'étendant en continu d'une bordure de coque à l'autre. La bande de scellage 4 s'étend aussi au-delà de ces bordures suivant une direction correspondant à ladite largeur de la bande et sensiblement parallèlement à l'axe de l'ouverture de chaque coque. Autrement dit, la bande s'étend de part et d'autre de chaque bordure d'ouverture pour déborder de chaque bordure en direction du fond de la coque correspondante afin d'assurer une bonne étanchéité de l'emballage au niveau des bordures des coques, c'est-à-dire sur toute la longueur de l'espace ou de la zone de contact définie entre les bordures et sur une largeur supérieure audit espace ou à ladite zone de contact.

La bande de scellage 4 d'un emballage 2, 3 recouvre ainsi la zone de séparation des coques dudit emballage qui forme un plan de joint entre les coques. Le plan de joint des coques d'un emballage correspond au plan moyen passant par les bordures de coques à l'état en appui d'une bordure contre l'autre.

Le fait de concevoir chaque emballage sous forme de deux coques définissant entre elle une chambre permet de n'avoir à sceller que la zone d'ouverture de ces coques en appliquant la bande autour de l'emballage le long des bordures de coque, ce qui permet de réduire la surface de scellage utilisée et l'encombrement de l'emballage tout en offrant une grande visibilité du contenu de l'emballage lorsque celui est transparent. En particulier, le contenu de l'emballage est visible sur 360° autour de l'axe de l'emballage, ainsi que par le dessus et le dessous, c'est-à-dire sur toute sa surface hormis la zone réduite de la surface de l'emballage occupée par la bande de scellage.

Comme rappelé ci-dessus, lorsque la bande de scellage d'un emballage 2, 3 est retirée ou déchirée, lesdites coques de l'emballage sont déplaçables l'une par rapport à l'autre en une position ouverte permettant d'accéder à l'intérieur dudit emballage, c'est-à-dire à l'intérieur des coques.

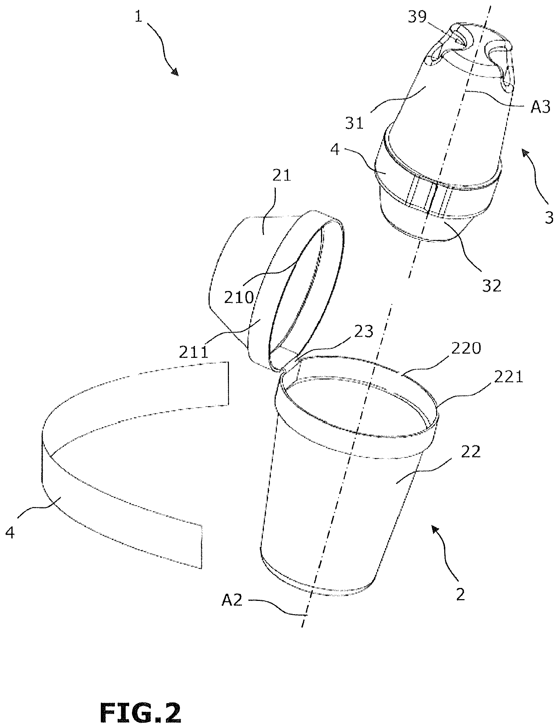

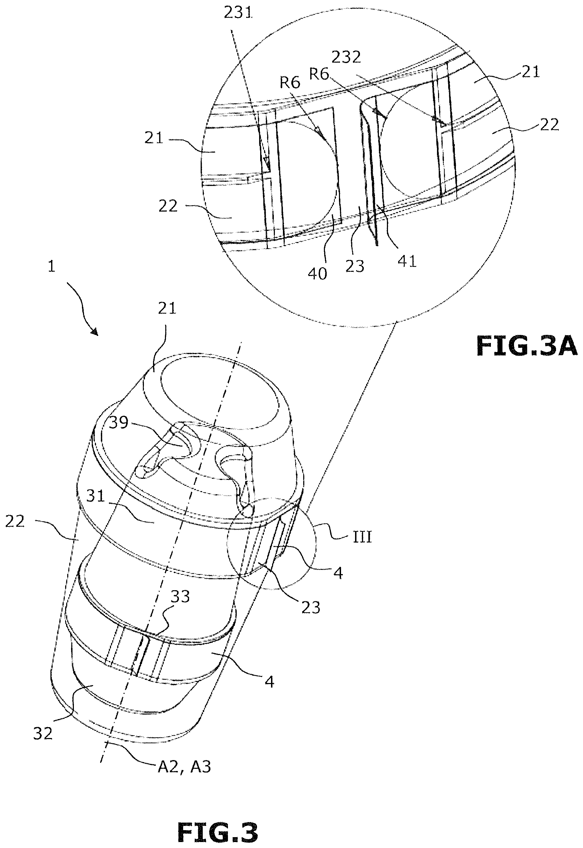

Dans l'exemple illustré aux figures 2 et 3, les deux coques 21, 22, 31, 32 de chaque emballage sont reliées entre elles par une charnière 23, 33. Les extrémités de ladite bande de scellage 4 recouvrent au moins les zones de la charnière 23, 33 qui jouxtent les bordures 210, 220 des coques. La zone de recouvrement de la bande sur chaque portion de charnière qui jouxte les bordures des coques s'étend sur un rayon R6 d'au moins 6mm pour répondre à la norme NF EN ISO 868 (en particulier -5) et EN ISO 11607 (en particulier -1).

La longueur de 6mm correspond à la longueur minimale de barrière qu'un liquide doit traverser pour entrer ou sortir de l'emballage au chemin le plus court, ce qui garantit une bonne étanchéité. Le centre 231, 232 du cercle de scellage de rayon R6 associé à chaque portion de charnière recouverte 23 correspond sensiblement à l'extrémité 231, 232 de la zone d'ouverture des coques située près de la portion de charnière correspondante.

Plus précisément, comme illustré plus particulièrement aux figures 3 et 3A, la bande de scellage 4 est appliquée sur la charnière 23 de l'emballage 2 correspondant par une première extrémité 40, puis est développée autour de l'emballage le long de la zone d'ouverture des coques et revient s'appliquer sur la charnière. La deuxième extrémité 41 de la bande peut être appliquée directement sur la charnière et/ou recouvrir la première extrémité 40 opposée de la bande. La description de l'application de la bande de scellage 4 à l'emballage 2 extérieur est aussi applicable à l'emballage 3 intérieur.

On obtient ainsi une très bonne étanchéité aux bactéries et au liquide, sans discontinuité le long de la zone d'ouverture des coques. A l'inverse, un chevauchement d'une extrémité de la bande par elle-même au niveau d'une partie de la zone d'ouverture de l'emballage, risquerait de provoquer une mauvaise étanchéité.

Avantageusement, la charnière 23, 33 d'un emballage 2, 3 est formée d'une seule pièce avec les deux coques de l'emballage. En pratique, la partie de ladite charnière qui relie les coques entre elles peut être formée par une zone de liaison déformable, par exemple en matière plastique. Ainsi, dans l'exemple illustré à la figure 2, la charnière 23 est formée dans la paroi de chaque coque 21, 22 de l'emballage 2.

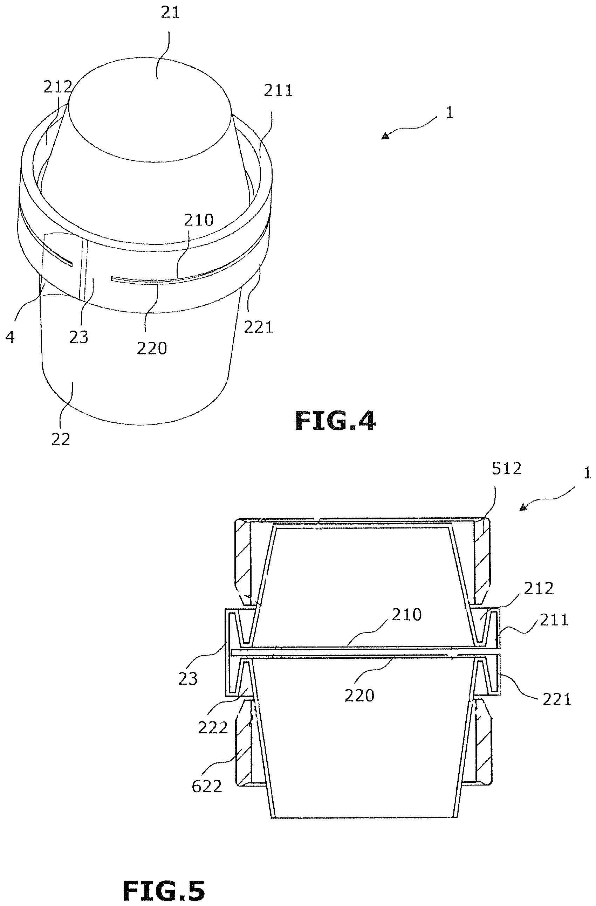

On peut aussi prévoir, comme illustré à la figure 5, que la charnière 23 est réalisée dans l'épaisseur des rebords 211, 221 (détaillés ci-après) qui prolongent les bordures 210, 220 des coques. En particulier, la face de la charnière 23 orientée du côté extérieur de l'emballage 2 s'étend dans la continuité de la face extérieure de chaque rebord. Dans ce cas le rebord qui prolonge chaque bordure se développe en continu tout le long de la coque y compris le long de la charnière. La charnière qui relie les deux coques entre elles s'étend alors dans l'encombrement des rebords qui prolongent les bordures des coques.

En variante, on peut prévoir que le rebord de chaque bordure de coque ne fasse que prolonger ladite bordure et s'arrête donc au niveau de la charnière entre les coques.

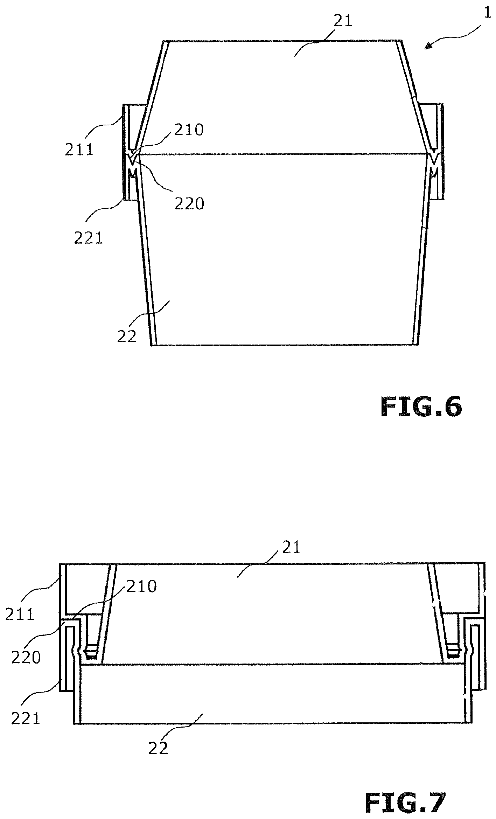

Les figures 6 et 7 illustrent des modes de réalisation d'emballage pour lesquels la bordure 210, 220, d'une coque présente une forme qui s'emboîte, c'est-à-dire complémentaire, avec la forme de la bordure 220, 210, de l'autre coque. L'emboitement des formes permet d'améliorer l'étanchéité et en particulier d'augmenter la longueur de la barrière qu'un éventuel liquide doit franchir.

Chaque bordure d'accès 210, 220 d'une coque 21, 22, 31, 32 est prolongée du côté extérieur de ladite coque 21, 22, 31, 32 par un rebord 211, 221. Ledit rebord 211, 221 se développe, par rapport à la zone d'ouverture de l'emballage définie par les bordure d'accès des coques, du côté de ladite coque 21, 22, 31, 32 et qui s'étend autour de la coque à écartement de la paroi extérieure de ladite coque 21, 22, 31, 32 pour ménager un espace 212, appelé espace d'insertion d'une pièce de contre-appui 512, 622, entre ledit rebord 211, 221 et la paroi extérieure de ladite coque 21, 22, 31, 32. La pièce de contre-appui 512, 622 peut être amovible ou peut restée liée à l'emballage.

Dans l'exemple illustré aux figures, chaque rebord 211, 221 présente une face extérieure sensiblement plane qui est parallèle à l'axe de l'ouverture de la coque correspondante emballage 2, 3. Ladite face extérieure de chaque rebord se développe sensiblement circulairement autour de l'emballage.

En variante, on peut prévoir que certains rebords ou les rebords forment avec l'axe A2, A3 de l'ouverture de la coque correspondante de l'emballage 2, 3 correspondant un angle donné non nul.

Avantageusement, chaque emballage 2, 3 est réalisé en matériau transparent, de préférence en matière thermoplastique ou en matière plastique moulée par exemple par injection. La transparence des emballages permet de déterminer aisément la nature de l'objet contenu dans l'emballage 3 intérieur.

La zone d'ouverture de l'emballage 3 intérieur, de même que celle de l'emballage 2 extérieur définie entre les bordures d'accès des coques est plus proche du fond d'une coque que du fond de l'autre coque dudit emballage.

Le décalage de la zone d'ouverture de chaque emballage 2, 3 par rapport au centre de l'emballage permet de privilégier l'orientation de présentation et d'ouverture de l'emballage 2, 3 correspondant. La grande coque 22 peut ainsi être tenue et/ou présentée à une hauteur inférieure à celle de la plus petite coque 21. En particulier, une telle conception de chaque emballage permet de définir pour l'utilisateur une coque inférieure destinée à former le fond de l'emballage pour retenir son contenu lorsque l'emballage est ouvert et une coque supérieure formant couvercle de fermeture de l'emballage. Le décentrage de la zone d'ouverture permet aussi de mieux visualiser le contenu de l'emballage.

L'emballage 3 intérieur comprend une zone de préhension 39 adaptée au positionnement d'au moins deux doigts d'une main. La zone de préhension 39 est ménagée à l'extrémité d'une des deux coques 31, 32 dudit emballage 3 intérieur. La zone de préhension 39 peut comprendre un ou des changements de section au niveau de la coque, par exemple par mise en forme en creux ou saillie du fond d'une coque. Dans l'exemple illustré aux figures 2 et 3, la zone de préhension 39 est ménagée sur la coque 31, qui s'étend en saillie de la coque 22 à l'état ouvert de l'emballage 2 extérieur.

Comme illustré aux figures, l'axe A3 de la zone d'ouverture de l'emballage 3 intérieur est sensiblement parallèle à l'axe A2 de la zone d'ouverture de l'emballage 2 extérieur. La zone d'ouverture de l'emballage extérieur 2 et la zone d'ouverture de l'emballage intérieur 3 sont décalées l'une de l'autre le long de l'axe A2 de la zone d'ouverture de l'emballage extérieur 2 ou encore le long de l'axe A3 de la zone d'ouverture de l'emballage 3 intérieur. Un tel décalage des zones d'ouvertures des emballages 2, 3 permet de dégager la visibilité de l'objet contenu dans la chambre de l'emballage 3 intérieur.

Pour chaque coque de chaque emballage, il est prévu une pièce 512, 622, de préférence de forme annulaire, appelée pièce de contre-appui ou contre-forme, apte à être insérée dans l'espace 212, 222 correspondant des coques 21, 22 de l'emballage, pour venir en appui de la paroi périphérique de la coque qui délimite l'espace intérieur de la coque et de la paroi du rebord 211, 221 orientée vers ladite paroi périphérique de la coque. Ainsi, les pièces de contre-appui 512, 622 insérées dans les espaces d'insertion 212, 222 de chaque coque d'un emballage permettent de rigidifier les rebords des coques pour appliquer avec pression et à chaud la bande 4 contre la face extérieure de chaque rebord en limitant le risque de déformation du rebord et de la coque correspondante.

Ainsi pour procéder au scellage d'un emballage 2 d'un double emballage tel que décrit ci-dessus, on positionne tout d'abord les bordures des coques de l'emballage en regard l'une de l'autre, si possible en contact l'une de l'autre afin de les sceller entre elles par une bande 4. Préférentiellement, avant l'application de la bande 4, on positionne une pièce de contre-appui 512, 622 dans l'espace 212, 222 d'insertion délimité entre la paroi périphérique de la coque 21, 22 et le rebord 211, 221 de chaque coque et, on applique la bande de scellage 4 sur les faces extérieures des rebords 211, 221 opposées aux pièces de contre-appui 512, 622. L'application de la bande de scellage 4 est réalisée à chaud et sous pression sur toute la longueur des bordures des coques. Les bordures des coques sont alors reliées entre elles par la même bande 4 qui s'étend le long de chacune des bordures et donc d'une bordure à l'autre, de sorte que la largeur de la bande recouvre les bordures des coques.

La bande peut être appliquée par un mouvement de rotation relative entre la bande et l'emballage, par exemple par application de galet(s) chauffé(s) sur la bande contre les rebords des coques.

Le recouvrement des rebords de coques par la bande entraine le recouvrement des bordures puisque les rebords prolongent lesdites bordures, la zone de séparation entre les coques étant définie par les bordures des deux coques.

Pour déconditionner l'objet contenu dans l'ensemble 1 et en particulier dans l'emballage 3 intérieur, on peut procéder de la manière suivante.

On entend par personne "sale" ou "contaminée" une personne travaillant dans des conditions non aseptisées, qui est susceptible de contaminer par ses mains les objets qu'elle touche. A l'inverse, on entend par personne "propre" une personne travaillant dans des conditions d'asepsie suffisante.

Une première personne, supposée en zone contaminée, retire la bande 4 de scellage de l'emballage 2 extérieur et déplace la coque 21 par rapport à la coque 22 par pivotement autour de la charnière 23 pour libérer l'accès à l'emballage 3 intérieur.

Ladite première personne « contaminée » peut ainsi tendre l'emballage 3 intérieur sans le toucher, en tenant l'emballage 2 extérieur par la coque inférieure 22. L'emballage 3 intérieur faisant saillie de la coque 22 de l'emballage 2 extérieur peut alors être saisi par une deuxième personne, supposée en zone propre. Après avoir saisi l'emballage 3 intérieur par la zone de préhension 39, ladite personne propre peut alors retirer la bande de scellage 4 de l'emballage 3 intérieur pour ouvrir ledit emballage en déplaçant la coque 32 supérieure par rapport à la coque 31 inférieure afin de saisir l'objet contenu dans ladite coque 31 inférieure. Ladite personne "propre" est par exemple le chirurgien au niveau du bloc opératoire. En effet la plus petite coque 32 est de préférence orientée en position supérieure pour former un couvercle par rapport à la coque 31 de plus grand volume qui est de préférence orientée en position inférieure pour retenir l'objet contenu dans ledit emballage 3 intérieur.

Ainsi, l'objet contenu dans l'emballage 3 intérieur n'a été ni touché ni lâché au cours de son déconditionnement. En outre, la chambre de l'emballage 3 intérieur contenant l'objet n'est ouverte qu'en zone propre.

## Revendications

1. Emballage (2, 3) médical comprenant deux coques (21, 22, 31, 32), chaque coque présentant une bordure (210, 220) délimitant une ouverture d'accès à l'intérieur de la coque, ledit emballage (2, 3) présentant au moins une configuration dite fermée selon laquelle les coques forment une chambre sensiblement fermée apte à loger un objet médical, tel qu'un implant médical, **caractérisé en ce que** ledit emballage (2 3) comprend aussi une bande de scellage (4) qui s'étend, du côté extérieur des coques (21, 22, 31, 32), sur toute la longueur des bordures (210, 220) des coques de l'emballage et d'une bordure (210, 220) à l'autre (220, 210) de manière à recouvrir les bordures afin de sceller la chambre délimitée par les deux coques,
et **en ce que**, les deux coques (21, 22, 31, 32) de l'emballage (2, 3) étant reliées entre elles par une charnière (23, 33), la bande de scellage (4) est appliquée par une (40) de ses extrémités (40, 41) sur la charnière (23, 33), s'étend autour de l'emballage (2, 3) le long des bordures des coques (21, 22, 31, 32) et revient s'appliquer sur la charnière (23, 33).

2. Emballage (2, 3) selon la revendication 1, **caractérisé en ce que** la zone de contact entre la bande de scellage (4) et les coques forme, le cas échéant avec la zone de contact d'une bordure (210, 220) de coque avec la bordure (220, 210) de l'autre coque, une zone de scellage dont le plus court chemin que doit parcourir un liquide pour traverser cette zone de scellage est au moins égal à 6 millimètres.

3. Emballage (2, 3) selon l'une des revendications précédentes, **caractérisé en ce que** la bordure (210, 220) d'une coque présente une forme qui s'emboite avec la forme de la bordure (220, 210) de l'autre coque.

4. Emballage (2, 3) selon l'une des revendications précédentes, **caractérisé en ce que** la bordure (210, 220) de chaque coque (21, 22, 31, 32) est prolongée du côté extérieur de ladite coque (21, 22, 31, 32) par un rebord (211, 221) qui se développe, par rapport à la zone d'ouverture de l'emballage définie entre les bordures des coques, du côté de ladite coque (21, 22, 31, 32) et qui s'étend à écartement de la paroi extérieure de ladite coque (21, 22, 31, 32) pour ménager un espace (212, 222), appelé espace d'insertion de pièce de contre-appui, entre ledit rebord (211, 221) et la paroi extérieure de ladite coque (21, 22, 31, 32).

5. Emballage (2, 3) selon la revendication 4, **caractérisé en ce que** chaque rebord (211, 221) présente une face extérieure, sensiblement plane, qui est soit parallèle à l'axe (A2, A3) de l'ouverture de la coque correspondante, soit forme avec l'axe (A2, A3) de l'ouverture de ladite coque un angle non nul.

6. Emballage (2, 3) selon l'une des revendications précédentes, **caractérisé en ce que** ledit emballage (2, 3) est réalisé en matériau transparent.

7. Emballage (2, 3) selon l'une des revendications précédentes, **caractérisé en ce que** la bande de scellage (4) est appliquée sur l'emballage (2, 3) à chaud et sous pression.

8. Emballage (2, 3) selon l'une des revendications précédentes, **caractérisé en ce que** la zone d'ouverture de l'emballage (2, 3), définie entre les bordures des coques dudit emballage, est plus proche du fond d'une coque que du fond de l'autre coque dudit emballage.

9. Emballage (3) selon l'une des revendications précédentes, **caractérisé en ce que** ledit emballage (3) comprend une zone de préhension (39) ménagée à l'extrémité d'une des deux coques (31, 32) dudit emballage (3).

10. Ensemble (1) comprenant deux emballages (2, 3) médicaux, dont un emballage (2), dit emballage extérieur, et un emballage dit emballage intérieur logé dans l'emballage extérieur, **caractérisé en ce que** chaque emballage (2, 3) est conforme à l'une des revendications précédentes.

11. Ensemble (1) selon la revendication 10, **caractérisé en ce que**, en configuration fermée des coques de chaque emballage, l'axe (A3) de l'ouverture des coques de l'emballage intérieur (3) est sensiblement parallèle à l'axe (A2) de l'ouverture des coques de l'emballage extérieur (2), la zone d'ouverture de l'emballage extérieur (2), définie entre les bordures de coques dudit emballage extérieur (2), et la zone d'ouverture correspondante de l'emballage intérieur (3) étant décalées l'une de l'autre le long de l'axe de l'ouverture des coques de l'emballage extérieur (2).

12. Procédé de scellage pour obtenir un emballage (2, 3) médical scellé conforme à la revendication 1, **caractérisé en ce que** ledit procédé de scellage comprend les étapes suivantes :
- fourniture d'un emballage médical comprenant deux coques (21, 22, 31, 32), chaque coque présentant une bordure (210, 220) délimitant une ouverture d'accès à l'intérieur de la coque, les coques étant aptes à présenter au moins une configuration dite fermée selon laquelle les coques forment une chambre sensiblement fermée apte à loger un objet médical, tel qu'un implant médical, les deux coques (21, 22, 31, 32) de l'emballage (2, 3) étant reliées entre elles par une charnière (23, 33),- positionnement des bordures (210, 220) des coques de l'emballage (2, 3) en regard l'une de l'autre, de préférence en contact l'une de l'autre,
- application d'une bande de scellage (4), du côté extérieur des coques (21, 22, 31, 32), sur toute la longueur des bordures (210, 220) des coques et d'une bordure (210, 220) à l'autre (220, 210) de manière à recouvrir les bordures afin de sceller la chambre délimitée par les deux coques,
la bande de scellage (4) étant appliquée par une (40) de ses extrémités (40, 41) sur la charnière (23, 33), s'étend autour de l'emballage (2, 3) le long des bordures des coques (21, 22, 31, 32) et revient s'appliquer sur la charnière (23, 33).

13. Procédé de scellage selon la revendication 12 pour obtenir un emballage (2, 3) scellé conforme à la revendication 4, **caractérisé en ce que**,
la bordure (210, 220) de chaque coque (21, 22, 31, 32) de l'emballage médical fourni étant prolongée du côté extérieur de ladite coque (21, 22, 31, 32) par un rebord (211, 221) qui se développe, par rapport à la zone d'ouverture de l'emballage définie entre les bordures des coques, du côté de ladite coque (21, 22, 31, 32) et qui s'étend à écartement de la paroi extérieure de ladite coque (21, 22, 31, 32) pour ménager un espace (212, 222), appelé espace d'insertion de pièce de contre-appui, entre ledit rebord (211, 221) et la paroi extérieure de ladite coque (21, 22, 31, 32),
le procédé comprend, avant l'application de la bande de scellage (4), le positionnement d'une pièce de contre-appui (512, 622) dans l'espace (212, 222) d'insertion correspondant de chaque coque, puis l'application de la bande de scellage (4) sur la face extérieure de chaque rebord (211, 221) opposée à la pièce de contre-appui correspondante.

## Patentansprüche

1. Medizinische Verpackung (2, 3), umfassend zwei Schalen (21, 22, 31, 32), wobei jede Schale einen Rand (210, 220) aufweist, der eine Zugangsöffnung in das Innere der Schale begrenzt, wobei die Verpackung (2, 3) mindestens eine Konfiguration, bezeichnet als geschlossen, aufweist, gemäß der die Schalen eine im Wesentlichen geschlossene Kammer bilden, die ausgelegt ist, um ein medizinisches Objekt wie z. B. ein medizinisches Implantat aufzunehmen, **dadurch gekennzeichnet, dass** die Verpackung (2, 3) auch ein Dichtungsband (4) umfasst, das sich, von der Außenseite der Schalen (21, 22, 31, 32) auf der gesamten Länge der Ränder (210, 220) der Schalen der Verpackung und von einem Rand (210, 220) zum anderen (220, 210) erstreckt, um die Ränder abzudecken, um die Kammer, begrenzt durch die zwei Schalen, abzudichten,
und dadurch, dass, da die zwei Schalen (21, 22, 31, 32) der Verpackung (2, 3) untereinander durch ein Scharnier (23, 33) verbunden sind, das Dichtungsband (4) durch eines (40) seiner Enden (40, 41) auf das Scharmier (23, 33) aufgebracht ist, sich um die Verpackung (2, 3) entlang der Ränder der Schalen (21, 22, 31, 32) erstreckt und erneut auf das Scharmier (23, 33) aufgebracht ist.

2. Verpackung (2, 3) nach Anspruch 1, **dadurch gekennzeichnet, dass** der Kontaktbereich zwischen dem Dichtungsband (4) und den Schalen gegebenenfalls mit dem Kontaktbereich eines Schalenrands (210, 220) mit dem Rand (220, 210) der anderen Schale einen Dichtungsbereich bildet, wobei der kürzeste Weg, den eine Flüssigkeit zurücklegen muss, um diesen Dichtungsbereich zu queren, mindestens gleich 6 Millimeter beträgt.

3. Verpackung (2, 3) nach einem der vorhergehenden Ansprüche, **dadurch gekennzeichnet, dass** der Rand (210, 220) einer Schale eine Form aufweist, die sich in die Form des Rands (220, 210) der anderen Schale einfügt.

4. Verpackung (2, 3) nach einem der vorhergehenden Ansprüche, **dadurch gekennzeichnet, dass** der Rand (210, 220) jeder Schale (21, 22, 31, 32) von der Außenseite der Schale (21, 22, 31, 32) durch eine Kante (211, 221) verlängert ist, die sich, mit Bezug auf den Öffnungsbereich der Verpackung, definiert zwischen den Rändern der Schalen, von der Seite der Schale (21, 22, 31, 32) entwickelt, und die sich in Abstand von der Außenwand der Schale (21, 22, 31, 32) erstreckt, um einen Raum (212, 222), bezeichnet als Raum zur Einführung eines Gegenstützteils, zwischen der Kante (211, 221) und der Außenwand der Schale (21, 22, 31, 32) anzubringen.

5. Verpackung (2, 3) nach Anspruch 4, **dadurch gekennzeichnet, dass** jeder Rand (211, 221) eine im Wesentlichen ebene Außenseite aufweist, die entweder parallel zur Achse (A2, A3) der Öffnung der entsprechenden Schale ist oder mit der Achse (A2, A3) der Öffnung der Schale einen Winkel von nicht null bildet.

6. Verpackung (2, 3) nach einem der vorhergehenden Ansprüche, **dadurch gekennzeichnet, dass** die Verpackung (2, 3) aus transparentem Material hergestellt ist.

7. Verpackung (2, 3) nach einem der vorhergehenden Ansprüche, **dadurch gekennzeichnet, dass** das Dichtungsband (4) auf die Verpackung (2, 3) heiß und unter Druck aufgebracht wird.

8. Verpackung (2, 3) nach einem der vorhergehenden Ansprüche, **dadurch gekennzeichnet, dass** der Öffnungsbereich der Verpackung (2, 3), definiert zwischen den Rändern der Schalen der Verpackung, näher am Boden einer Schale als am Boden der anderen Schale der Verpackung ist.

9. Verpackung (3) nach einem der vorhergehenden Ansprüche, **dadurch gekennzeichnet, dass** die Verpackung (3) einen Greifbereich (39) umfasst, der am Ende einer der zwei Schalen (31, 32) der Verpackung (3) angeordnet ist.

10. Einheit (1), umfassend zwei medizinische Verpackungen (2, 3), davon eine Verpackung (2), bezeichnet als äußere Verpackung, und eine Verpackung, bezeichnet als innere Verpackung, die in der äußeren Verpackung untergebracht ist, **dadurch gekennzeichnet, dass** jede Verpackung (2, 3) nach einem der vorhergehenden Ansprüche ist.

11. Anordnung (1) nach Anspruch 10, **dadurch gekennzeichnet, dass** in der geschlossenen Konfiguration der Schalen jeder Verpackung die Achse (A3) der Öffnung der Schalen der inneren Verpackung (3) im Wesentlichen parallel zur Achse (A2) der Öffnung der Schalen der äußeren Verpackung (2) ist, wobei der Öffnungsbereich der äußeren Verpackung (2), definiert zwischen den Schalenrändern der äußeren Verpackung (2), und der entsprechende Öffnungsbereich der inneren Verpackung (3) voneinander entlang der Achse der Öffnung der Schalen der äußeren Verpackung (2) versetzt sind.

12. Dichtungsverfahren zum Erhalten einer medizinischen Verpackung (2, 3), die gemäß Anspruch 1 abgedichtet ist, **dadurch gekennzeichnet, dass** das Dichtungsverfahren die folgenden Schritte umfasst:
- Bereitstellen einer medizinischen Verpackung, umfassend zwei Schalen (21, 22, 31, 32), wobei jede Schale einen Rand (210, 220) darstellt, der eine Öffnung zum Zugang in das Innere der Schale begrenzt, wobei die Schalen ausgelegt sind, um mindestens eine Konfiguration, bezeichnet als geschlossen, darzustellen, gemäß der die Schalen eine im Wesentlichen geschlossene Kammer bilden, die ausgelegt ist, um ein medizinisches Objekt wie z. B. ein medizinisches Implantat aufzunehmen, wobei die zwei Schalen (21, 22, 31, 32) der Verpackung (2, 3) untereinander durch ein Scharnier (23, 33) verbunden sind,
- Positionieren der Ränder (210, 220) der Schalen der Verpackung (2, 3) einander gegenüber, vorzugsweise in Kontakt miteinander.
- Anbringen eines Dichtungsbands (4) auf der Außenseite der Schalen (21, 22, 31, 32), auf der gesamten Länge der Ränder (210, 220) der Schalen und von einem Rand (210, 220) zum andern (220, 210), um die Ränder abzudecken, um die Kammer, begrenzt von den zwei Schalen, abzudichten.
wobei das Dichtungsband (4) durch eines (40) seiner Enden (40, 41) auf das Scharnier (23, 33) aufgebracht ist, sich um die Verpackung (2, 3) entlang der Ränder der Schalen (21, 22, 31, 32) erstreckt und erneut auf das Scharnier (23, 33) aufgebracht wird.

13. Dichtungsverfahren nach Anspruch 12 zum Erhalten einer abgedichteten Verpackung (2, 3) gemäß Anspruch 4, **dadurch gekennzeichnet, dass**
der Rand (210, 220) jeder Schale (21, 22, 31, 32) der bereitgestellten medizinischen Verpackung auf der Außenseite der Schale (21, 22, 31, 32) durch einen Rand (211, 221) verlängert ist, der sich mit Bezug auf den Öffnungsbereich der Verpackung, definiert zwischen den Rändern der Schalen, von der Seite der Schale (21, 22, 31, 32) entwickelt, und der sich, in Abstand von der Außenwand der Schale (21, 22, 31, 32) erstreckt, um einen Raum (212, 222), bezeichnet als Raum zur Einführung eines Gegenstützteils, zwischen dem Rand (211, 221) und der Außenwand der Schale (21, 22, 31, 32) anzuordnen,
das Verfahren, vor dem Aufbringen des Dichtungsbands (4), das Positionieren eines Gegenstützteils (512, 622) im Einführungsraum (212, 222) umfasst, der jeder Schale entspricht, dann das Aufbringen des Dichtungsbands (4) auf der Außenseite jedes Rands (211, 221) gegenüber dem entsprechenden Gegenstützteil umfasst.

## Claims

1. Medical packaging (2, 3) comprising two shells (21, 22, 31, 32), each shell having a border (210, 220) delimiting an opening for access to the inside of the shell, said packaging (2, 3) having at least one configuration, called closed configuration, in which the shells form a substantially closed chamber capable of housing a medical object, such as a medical implant, **characterized in that** said packaging (2, 3) also comprises a sealing strip (4) that extends, on the outer side of the shells (21, 22, 31, 32), over the entire length of the borders (210, 220) of the shells of the packaging and from one border (210, 220) to the other (220, 210) so as to cover the borders in order to seal the chamber delimited by the two shells,
and **in that**, the two shells (21, 22, 31, 32) of the packaging (2, 3) being connected to each other by a hinge (23, 33), the sealing strip (4) is applied by one (40) of its ends (40, 41) on the hinge (23, 33), extends around the packaging (2, 3) along the borders of the shells (21, 22, 31, 32) and returns to be applied on the hinge (23, 33).

2. The packaging (2, 3) according to claim 1, **characterized in that** the contact zone between the sealing strip (4) and the shells forms, if applicable with the contact zone of one shell border (210, 220) with the border (220, 210) of the other shell, a sealing zone, the shortest path of which that a liquid must travel to pass through this sealing zone being at least equal to 6 millimeters.

3. The packaging (2, 3) according to one of the preceding claims, **characterized in that** the border (210, 220) of a shell has a shape that nests with the shape of the border (220, 210) of the other shell.

4. The packaging (2, 3) according to one of the preceding claims, **characterized in that** the border (210, 220) of each shell (21, 22, 31, 32) is extended on the outer side of each shell (21, 22, 31, 32) by a rim (211, 221) that develops, relative to the opening zone of the package defined between the borders of the shells, on the side of said shell (21, 22, 31, 32) and that extends away from the outer wall of said shell (21, 22, 31, 32) to arrange a space (212, 222), called counter-bearing piece insertion space, between said rim (211, 221) and the outer wall of said shell (21, 22, 31, 32).

5. The packaging (2, 3) according to claim 4, **characterized in that** each rim (211, 221) has a substantially planar outer face that is either parallel to the axis (A2, A3) of the opening of the corresponding shell, or that forms a nonzero angle with the axis (A2, A3) of the opening of said shell.

6. The packaging (2, 3) according to one of the preceding claims, **characterized in that** said packaging (2, 3) is made from transparent material.

7. The packaging (2, 3) according to one of the preceding claims, **characterized in that** the sealing strip (4) is applied on the packaging (2, 3) hot and with pressure.

8. The packaging (2, 3) according to one of the preceding claims, **characterized in that** the opening zone of the packaging (2, 3), defined between the borders of the shells of said packaging, is closer to the bottom of one shell than the bottom of the other shell of said packaging.

9. The packaging (3) according to one of the preceding claims, **characterized in that** said packaging (3) comprises a gripping zone (39) arranged at the end of one of the two shells (31, 32) of said packaging (3).

10. An assembly (1) comprising two medical packaging items (2, 3), including a packaging item (2) called outer packaging, and a packaging item called inner packaging housed in the outer packaging, **characterized in that** each packaging item (2, 3) is according to one of the preceding claims.

11. The assembly (1) according to claim 10, **characterized in that**, in the closed configuration of the shells of each packaging item, the axis (A3) of the opening of the shells of the inner packaging (3) is substantially parallel to the axis (A2) of the opening of the shells of the outer packaging (2), the opening zone of the outer packaging (2), defined between the shell borders of said outer packaging (2), and the corresponding opening zone of the inner packaging (3) being offset relative to one another along the opening axis of the shells of the outer packaging (2).

12. A sealing method in order to obtain sealed medical packaging (2, 3) according to claim 1, **characterized in that** said sealing method comprises the following steps:
- providing a medical packaging item comprising two shells (21, 22, 31, 32), each shell having a border (210, 220) delimiting an access opening to the inside of the shell, the shells being able to have at least one configuration, called closed configuration, in which the shells form a substantially closed chamber able to house a medical object, such as a medical implant, the two shells (21, 22, 31, 32) of the packaging (2, 3) being connected to each other by a hinge (23, 33),
- positioning borders (210, 220) of the shells of the packaging (2, 3) opposite each other, preferably in contact with each other,
- applying a sealing strip (4), on the outer side of the shells (21, 22, 31, 32), over the entire length of the borders (210, 220) of the shells and from one border (210, 220) to the other (220, 210) so as to cover the borders in order to seal the chamber delimited by the two shells,
the sealing strip (4) being applied by one (40) of its ends (40, 41) on the hinge (23, 33), extends around the packaging (2, 3) along the borders of the shells (21, 22, 31, 32) and becomes applied on the hinge (23, 33).

13. The sealing method according to claim 12 in order to obtain a sealed packaging item (2, 3) according to claim 4, **characterized in that**,
the border (210, 220) of each shell (21, 22, 31, 32) of the provided medical packaging item being extended on the outer side of said shell (21, 22, 31, 32) by a rim (211, 221) that develops, relative to the opening zone of the packaging defined between the borders of the shells, on the side of said shell (21, 22, 31, 32) and that extends away from the outer wall of said shell (21, 22, 31, 32) to arrange a space (212, 222), called counter-bearing piece insertion space, between said rim (211, 221) and the outer wall of said shell (21, 22, 31, 32),
the method comprises, before the application of the sealing strip (4), positioning a counter-bearing piece (512, 622) in the corresponding insertion space (212, 222) of each shell, then applying the sealing strip (4) on the outer face of each rim (211, 221) opposite the corresponding counter-bearing piece.
